# EUROPÄISCHE PATENTSCHRIFT

(11) **EP 0 739 892 B1**
(45) Veröffentlichungstag und Bekanntmachung des Hinweises auf die Patenterteilung: **25.09.2002**
(21) Anmeldenummer: 96810237.6
(22) Anmeldetag: 16.04.1996
(51) Int. Cl.: C07D 405/12, A61K 31/445, A61K 31/35

(54) **Chromon-Derivate**
Chromone derivatives
Dérivés de chromone

(30) Priorität: 24.04.1995 CH 115795
(43) Veröffentlichungstag der Anmeldung: 30.10.1996
(73) Patentinhaber: Novartis AG, 4056 Basel (CH); Novartis-Erfindungen Verwaltungsgesellschaft m.b.H., 1235 Wien (AT)
(72) Erfinder: von Sprecher, Andreas, Dr., 4104 Oberwil (CH); Gerspacher, Marc, Dr., 5073 Gipf-Oberfrick (CH); Mah, Robert, Dr., 4123 Allschwil (CH); Schilling, Walter, Dr., 4204 Himmelried (CH); Ofner, Silvio, Dr., 4142 Münchenstein (CH); Veenstra, Siem Jacob, Dr., 4053 Basel (CH); Roggo Silvio, CH-4132 Muttenz (CH)

(56) Entgegenhaltungen:
- EP-A- 0 532 456
- WO-A-96/10562

## Beschreibung

Die Erfindung betrifft die Verbindungen der Formel I, 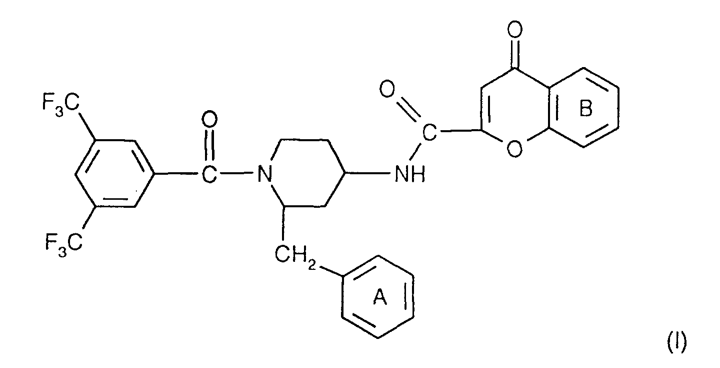 worin der Ring A unsubstituiert oder substituiert ist und der Ring B unsubstituiert oder substituiert ist. Diese Verbindungen weisen wertvolle pharmakologische Eigenschaften auf und sind insbesondere als Neurokinin 1-Antagonisten bzw. Substanz P-Antagonisten wirksam.

Insbesondere betrifft die Erfindung die Verbindungen der Formel I, worin der Ring A unsubstituiert oder durch Niederalkyl, Niederalkoxy, Halogen, Nitro oder Trifluormethyl monosubstituiert ist, und worin der Ring B unsubstituiert oder durch 1-4 Substituenten ausgewählt aus der Gruppe bestehend aus Niederalkyl, Hydroxy, Niederalkoxy, Niederalkylthio, Halogen, Nitro, Cyano und Trifluormethyl substituiert ist, und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Da die erfindungsgemässen Verbindungen mindestens zwei optisch aktive Kohlenstoffatome aufweisen, können sie dementsprechend in Form von Stereoisomeren, Stereoisomerengemischen sowie in Form der (im wesentlichen) reinen Diastereomeren vorliegen. Entsprechende Stereoisomere sind ebenfalls von der vorliegenden Erfindung umfasst. Bevorzugt liegen die Verbindungen der Formel I in der diastereomeren Form vor, wie sie in Formel la wiedergegeben ist:

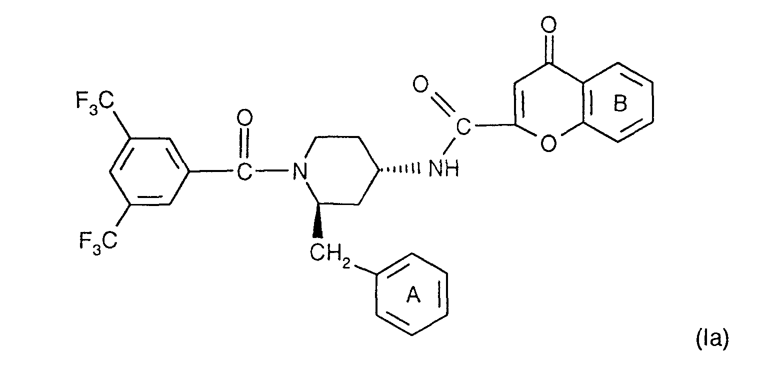

Die vor- und nachstehend verwendeten Allgemeinbegriffe haben, sofern nicht abweichend definiert, die nachfolgend angegebenen Bedeutungen.

Der Ausdruck "nieder" bedeutet, dass entsprechende Gruppen und Verbindungen jeweils 1 bis und mit 7, vorzugsweise 1 bis und mit 4, C-Atome enthalten.

Niederalkyl ist z.B. Methyl, Ethyl, n-Propyl, Isopropyl, n-Butyl, Isobutyl, sek-Butyl, tert-Butyl oder ein entsprechender Pentyl-, Hexyl- oder Heptylrest. Bevorzugt ist C₁-C₄-Alkyl.

Niederalkoxy ist z.B. Methoxy, Ethoxy, n-Propyloxy, lsopropyloxy, n-Butyloxy, Isobutyloxy, sec-Butyloxy, tert-Butyloxy oder ein entsprechender Pentyloxy-, Hexyloxy-, oder Heptyloxyrest. Bevorzugt ist C₁-C₄-Alkoxy.

Halogen ist z.B. Fluor, Chlor oder Brom, kann aber auch lod bedeuten. Bevorzugt ist Chlor.

Die Verbindungen der Formel I können als, insbesondere pharmazeutisch verwendbare, Salze vorliegen. Mit dem basischen Zentrum des Piperidinrings können Säureadditionssalze gebildet werden. Als Säurekomponente kommen beispielsweise starke anorganische Säuren, wie Mineralsäuren, z.B. Schwefelsäure, Phosphorsäuren, z.B. Orthophosphorsäure, Halogenwasserstoffsäuren, z.B. Chlorwasserstoffsäure, oder starke organische Carbonsäuren, wie gegebenenfalls, z.B. durch Halogen, substituierte Niederalkancarbonsäuren, z.B. Essigsäure oder Trifluoressigsäure, gegebenenfalls ungesättigte Dicarbonsäuren, z.B. Oxal-, Malon-, Bernstein-, Malein-, Fumar-, Phthal- oder Terephthalsäure, Hydroxycarbonsäuren, z.B. Ascorbin-, Glykol-, Milch-, Äpfel-, Wein- oder Zitronensäure, Aminosäuren, z.B. Asparagin- oder Glutaminsäure, oder Benzoesäure, oder organische Sulfonsäuren, wie gegebenenfalls, z.B durch Halogen, substituierte Niederalkansulfonsäuren, z.B. Methansulfonsäure, oder gegebenenfalls, z.B. durch Niederalkyl, substituierte Arylsulfonsäuren, z.B. p-Toluolsulfonsäure, in Betracht. Umfasst sind ferner für die therapeutische Verwendung nicht geeignete Salze, die beispielsweise für die Isolierung bzw. Reinigung von freien Verbindungen der Formel I oder deren pharmazeutisch verwendbaren Salzen eingesetzt werden können. Zur therapeutischen Anwendung gelangen nur die pharmazeutisch verwendbaren, nicht-toxischen Salze, die deshalb bevorzugt sind.

Die Verbindungen der Formel I - einschliesslich ihrer pharmazeutisch verwendbaren Salze, die im folgenden jeweils mit eingeschlossen sind - haben wertvolle pharmakologische Eigenschaften. Insbesondere wirken sie als Neurokinin 1-Antagonisten (NK1-Antagonisten) und sind daher in der Lage, Krankheitserscheinungen zu verhindern, die u.a. durch Ausschüttung von Substanz P ausgelöst werden.

Die Atemwege sind mit sensorischen Nerven ausgestattet, die eine Reihe von Neuropeptiden enthalten, insbesondere Tachykinine und CGRP ( = Calcitonin gene-related peptide). Die Aktivierung der sensorischen Nerven führt zu einer lokalen Freisetzung von Neuropeptiden innerhalb der Lunge. Ausgeschüttet werden vor allem Substanz P und Neurokinin A, die eine akute entzündliche Reaktion auslösen, die als "neurogenic inflammation" bezeichnet wird. Diese entzündliche Reaktion läuft hauptsächlich via NK1-Rezeptor-Aktivierung ab und umfasst insbesondere Vasodilatation, mikrovaskuläre Lecke, Rekrutierung von inflammatorischen Leukozyten und übermässige Schleim-Absonderungen. Diese Substanz P-Effekte sind typische Merkmale von Asthma.

Die pharmakologische Wirkung der Verbindungen der Formel I beruht insbesondere auf der Antagonisierung des NK1-Rezeptors. Die Verbindungen der Formel I sind also in der Lage, die "neurogenic inflammation" sowie die Tachykinin-induzierte Bronchokonstriktion zu hemmen.

Die vorteilhaften Wirkungen der Verbindungen der Formel I lassen sich durch verschiedene in vitro oder in vivo Testmethoden nachweisen. So sind sie z.B. in vivo im NK1-Bronchospasmustest am Meerschweinchen mit ED50-Werten ab ca. 0.03 mg/kg p.o. wirksam, wobei die Testsubstanzen 2, 4 oder 24 Stunden vor der intravenösen Gabe von 3.0 µg/kg [Sar9,Met(O2)11]-Substanz P [ = SarSP] verabreicht werden. Durch den "Challenge" mit SarSP wird beim Meerschweinchen eine Erhöhung des intratrachealen Drucks induziert. Die erfindungsgemässen Verbindungen zeichnen sich durch eine extrem gute orale Wirksamkeit sowie eine ungewöhnlich lange Wirkungsdauer aus.

Als Antagonisten der NK1-Rezeptoren sind die Verbindungen der Formel I therapeutisch nützlich bei der Vorbeugung, der Behandlung oder der Diagnose einer Anzahl von Krankheiten, zum Beispiel: Krankheiten der oberen und unteren Atemwege, wie z.B. Bronchialasthma, allergisches Asthma, nicht-allergisches Asthma, allergische Ueberempfindlichkeiten und Hypersekretionszustände wie chronische Bronchitis und cystische Fibrose; Lungenfibrose unterschiedlicher Aetiologie; Krankheiten des Lungenund Bronchial-Kreislaufes, wie Lungen-Bluthochdruck, Angiogenese, Metastasen; Krankheiten des Magen-Darmtraktes, wie Morbus Crohn, Morbus Hirsprung, Diarrhö, Malabsorptionszustände, Entzündungszustände; bei affektiven, traumatischen oder entzündlichen Störungen des zentralen und peripheren Nervensystems wie Depressionen, Angstzuständen, Migräne und anderen Formen von kranialen Schmerzen, Schlaganfällen, Emesis; Krankheiten der Blutgefässe, wie der kranialen Gefässe; Krankheiten, welche die Mikrozirkulation in diversen Geweben wie Haut und Augen betreffen; Krankheiten des Immun- und des Retikulohistiozytären Systems wie in Milz- und lymphatischen Geweben; Schmerzzustände und andere Krankheiten, bei denen die Wirkung von Neurokininen, Tachykininen oder anderen verwandten Substanzen an der Pathogenese, Pathologie und Aetiologie beteiligt sind.

Substanz P ist ein natürlich vorkommendes Undekapeptid der Tachykininfamilie. Es wird im Säugetierorganismus erzeugt und wirkt pharmakologisch als Neuropeptid. Substanz P spielt eine wesentliche Rolle bei verschiedenen Erkrankungen, beispielsweise bei Schmerzzuständen, bei Migräne und bei einigen Störungen des Zentralnervensystems, wie bei Angstzuständen, Emesis, Schizophrenie und Depressionen sowie bei bestimmten motorischen Störungen, wie bei morbus Parkinson, aber auch bei entzündlichen Erkrankungen, wie bei rheumatoider Arthritis, Iritis und Konjunktivitis, bei Erkrankungen der Atmungsorgane, wie bei Asthma und chronischer Bronchitis, bei Erkrankungen des Gastrointestinalsystems, wie bei ulcerativer Colitis und Morbus Crohn, und bei Hypertension.

Das Ziel vielfacher Bemühungen ist es, die Entwicklung auf dem Gebiet der Substanz P-Antagonisten weiterzutreiben und beispielsweise geeignete Substanz P-Antagonisten mit einem breiteren Wirkungsspektrum zu finden, die eine erhöhte Aktivität und eine erhöhte Bioverfügbarkeit sowie eine verbesserte chemische Stabilität und Kristallinität aufweisen.

Ausgedehnte pharmakologische Untersuchungen haben ergeben, dass die erfindungsgemässen Verbindungen und ihre Salze in besonders bevorzugtem Masse Substanz P antagonisieren und somit in besonderer Weise zur Behandlung der durch Substanz P ausgelösten Krankheitserscheinungen geeignet sind.

Die Substanz P-antagonisierenden Effekte können - z.B. wie nachstehend dargelegt - unter Heranziehung von Testmethoden, die dem Fachmann bekannt sind, nachgewiesen werden. Derartige Effekte werden sowohl *in vitro* als auch *in vivo* gefunden. So wird durch die Verbindungen der Formel I z.B. die Bindung von ³H-Substanz P an der Rinder-Retina im Radiorezeptor-Assay nach H. Bittiger, Ciba Foundation Symposium 91 (1982) 196-199 in unerwartet starker Weise mit IC₅₀-Werten ab etwa 5 nM gehemmt.

Weiterhin wird z.B. *in vitro* die durch Substanz P induzierte Bildung von Phosphoinositol in menschlichen Astrocytoma-Zellen antagonisiert. Dabei ergeben sich IC₅₀-Werte ab etwa 1 nM. Als Testmodell für den Nachweis dieser Hemmung eignet sich beispielsweise die Testmethode von C.M. Lee et al., wie sie in J. Neurochem. 59 (1992) 406-414 beschrieben wird.

Durch i.c.v.-Verabreichung von Substanz P-methylester bei Wüstenspringmäusen (gerbil) wird eine Verhaltensänderung hervorgerufen. Dieser Effekt kann nach peroraler Verabreichung von Verbindungen der Formel I *in vivo* gehemmt werden. Als Testmethodik wird das Verfahren von A.Vassout et al. verwendet, welches am Kongress "Substance P and Related Peptides: Cellular and Molecular Physiology" in Worchester, Mass. 1990 vorgestellt wurde. Dabei werden ED₅₀-Werte ab etwa 0.1 mg/kg p.o. ermittelt. Aus diesen Daten ergibt sich die hervorragende Eignung der Verbindungen der Formel I für die Behandlung von Erkrankungen des zentralen Nervensystems.

Gegenüber den bisher bekannten NK1- bzw. Substanz P-Antagonisten besitzen die erfindungsgemässen Verbindungen eine deutlich höhere Aktivität, ferner weisen sie auch eine wesentlich höhere chemische Stabilität, z.B. gegenüber Sauerstoff, eine verbesserte Kristallinität sowie eine gesteigerte orale Bioverfügbarkeit auf.

Die erfindungsgemäss bereitgestellten Substanz P-Antagonisten der Formel I eignen sich dementsprechend vorzüglich zur therapeutischen Behandlung der vorstehend aufgeführten pathologischen Erscheinungen.

Ganz besonders betrifft die Erfindung die Verbindungen der Formel I, worin der Ring A unsubstituiert oder durch Halogen monosubstituiert ist, und worin der Ring B unsubstituiert oder durch 1-2 Substituenten ausgewählt aus der Gruppe bestehend aus Niederalkyl, Hydroxy, Niederalkoxy, Niederalkylthio, Halogen, Nitro und Cyano substituiert ist, und pharmazeutisch anwendbare Salze davon.

In erster Linie betrifft die Erfindung die Verbindungen der Formel I, worin der Ring A unsubstituiert oder durch Chlor monosubstituiert ist, und worin der Ring B unsubstituiert oder durch 1-2 Substituenten ausgewählt aus der Gruppe bestehend aus Hydroxy, Niederalkoxy, Chlor und Brom substituiert ist, und pharmazeutisch anwendbare Salze davon.

Vor allen Dingen betrifft die Erfindung die Verbindungen der Formel I, worin der Ring A unsubstituiert oder durch Chlor monosubstituiert ist, und worin der Ring B unsubstituiert oder durch Chlor oder Fluor monosubstituiert ist, und pharmazeutisch anwendbare Salze davon.

Als Untergruppen aus einer Gruppe von Verbindungen der Formel I sind jeweils hervorzuheben: (a) Verbindungen der Formel I, worin der Ring A unsubstituiert oder in 4-Stellung durch Chlor substituiert ist; (b) Verbindungen der Formel I, worin der Ring A in 4-Stellung durch Chlor substituiert ist; (c) Verbindungen der Formel I, worin der Ring B durch Chlor oder Fluor monosubstituiert ist; (d) Verbindungen der Formel I, worin der Ring B unsubstituiert ist.

Die Erfindung betrifft vor allem die in den Beispielen beschriebenen spezifischen Verbindungen und Salze, insbesondere pharmazeutisch anwendbare Salze, davon.

Die Verbindungen der Formel I können in an sich bekannter Weise hergestellt werden, indem man z.B.
a) eine Verbindung der Formel IIa, 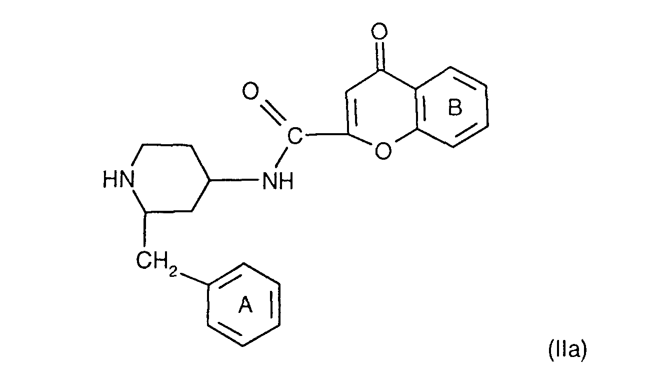 worin die Ringe A und B wie unter Formel I definiert sind, mit einer Verbindung der Formel IIb, 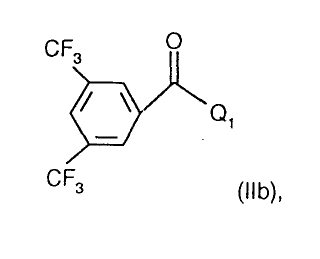 worin Q₁ gegebenenfalls verethertes Hydroxy, wie Hydroxy, Niederalkoxy oder gegebenenfalls substituiertes Phenoxy, oder reaktionsfähiges verestertes Hydroxy, wie Halogen, insbesondere Chlor, oder einen Rest der Formel 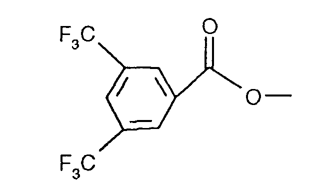 bedeutet, oder einem Salz davon, umsetzt; oder
b) eine Verbindung der Formel IIIa, 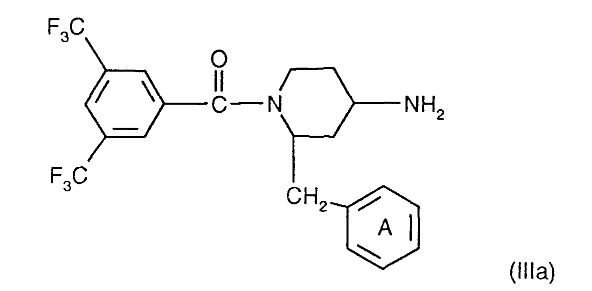
worin der Ring A wie unter Formel I angegeben definiert ist, mit einer Verbindung der Formel IIIb, 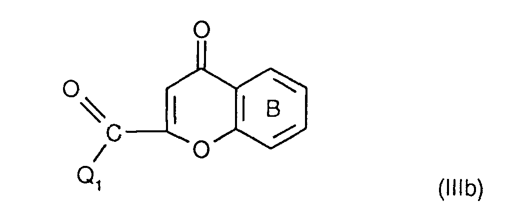 worin der Ring B wie unter Formel I angegeben definiert ist und Q₁ gegebenenfalls verethertes Hydroxy, wie Hydroxy, Niederalkoxy oder gegebenenfalls substituiertes Phenoxy, oder reaktionsfähiges verestertes Hydroxy, wie Halogen, insbesondere Chlor, oder einen Rest der Formel 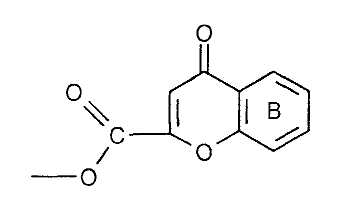 bedeutet, oder einem Salz davon, umsetzt;
und, wenn erwünscht, eine Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, eine erhaltene Mischung von Stereoisomeren oder Diastereomeren in die einzelnen Stereoisomeren bzw. Diastereomeren auftrennt.

Salze von Ausgangsmaterialien, die mindestens ein basisches Zentrum aufweisen, beispielsweise der Formel lla oder IIIa, sind entsprechende Säureadditionssalze, während Salze von Ausgangsstoffen, die eine acide Gruppe aufweisen, beispielsweise der Formel IIb oder IIIb, als Salze mit Basen vorliegen.

In der folgenden näheren Beschreibung der Verfahren haben die Ringe A und B jeweils die unter Formel I angegebene Bedeutung, sofern nichts anderes angegeben ist.

Die vor- und nachstehend in den Varianten beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80°C bis zur Siedetemperatur des Reaktionsmediums, vorzugsweise von etwa -10° bis etwa +200°C, und, falls erforderlich, in einem geschlossenen Gefäss, unter Druck, in einer Inertgasatmosphäre und/oder unter wasserfreien Bedingungen arbeitet.

Verfahrensvarianten a) und b): Die Kondensation zur Herstellung der jeweiligen Amidbindung kann in an sich bekannter Weise durchgeführt werden, beispielsweise wie in Standardwerken, wie "Houben-Weyl, Methoden der organischen Chemie", 4. Auflage, Band 15/11, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Principles of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3'sulfonat und 2-tert-Butyl-5-methylisoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoramidochloridat, Bis-(2-oxo-3-oxazolidinyl)-phosphinsäurechlorid oder 1-Benzotriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. Ethyldiisopropylamin, oder eine heterocyclische Base, z.B. Pyridin, 4-Dimethylaminopyridin oder bevorzugt N-Methylmorpholin.

Die Kondensation eines Carbonsäurehalogenids beispielsweise mit einem entsprechenden Amin kann auch in Gegenwart einer geeigneten Base ohne Zusatz einer geeigneten Kupplungskomponente erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, einem cyclischen Ether, z.B. Tetrahydrofuran, einem Ester, z.B. Essigsäureethylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden.

Die Ausgangsmaterialien der Formel llb und IIIb sind bekannt oder können in an sich bekannter Weise hergestellt werden.

Verbindungen der Formel IIIa können in an sich bekannter Weise hergestellt werden. Beispielsweise geht man von einer Verbindung der Formel IIIc aus, 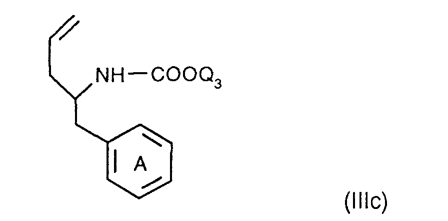 worin Q₃ z.B. Niederalkyl oder Phenylniederalkyl bedeutet. Diese wird z.B. durch Umsetzung mit Niederalkoxy-halogen-methan, wie Ethoxy-chlormethan, in Gegenwart einer Base N-alkyliert. Die so erhältliche Verbindung der Formel (IIId), 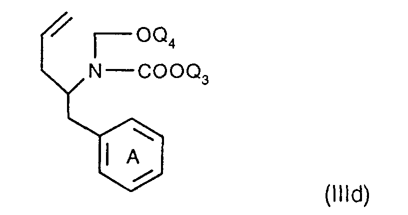 worin Q₄ z.B. Niederalkyl bedeutet, behandelt man mit einem Nitril, z.B. Acetonitril, in Gegenwart einer starken Säure, z.B. Chlorsulfonsäure. In der so resultierenden Verbindung der Formel IIIe 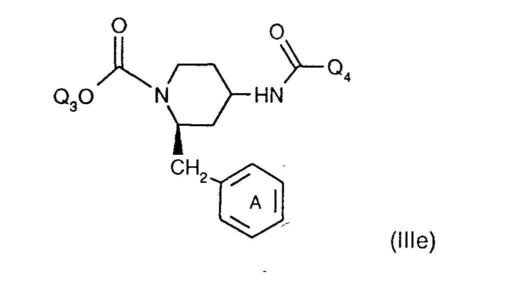 wird die -C(=O)-OQ₃-Gruppe durch Behandeln mit einer starken Säure, z.B. Bromwasserstoffsäure, abgespalten.

Zur Herstellung einer enantiomerenreinen Verbindung wird in einer so erhältlichen Verbindung der Formel IIIf 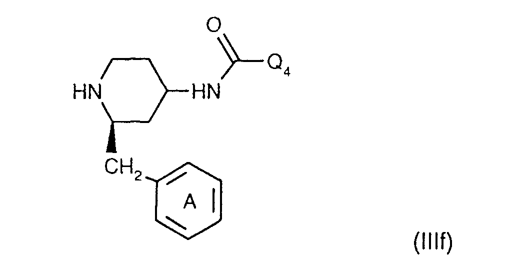 die sekundäre Aminogruppe mit einer optisch aktiven Verbindung, wie einer entsprechenden O-acylierten α-Hydroxycarbonsäure oder einem reaktionsfähigen Derivat davon, z.B. O-Acetyl-(+)mandelsäure-chlorid, acyliert und das so zugängliche Diastereomerengemisch in an sich bekannter Weise, z.B. chromatographisch, aufgespalten. Nach Abspaltung beider N-Acylgruppen, beispielsweise duch saure Hydrolyse, z.B. mit Salzsäure, erhält man eine Verbindung der Formel IIIg:

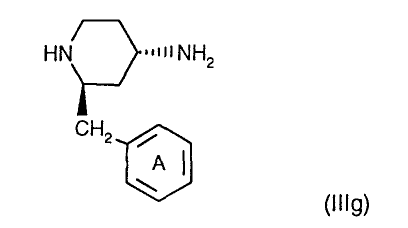

Die 4-Aminogruppe von Verbindungen der Formel IIIg wird vorübergehend in an sich bekannter Weise geschützt, beispielsweise durch Umsetzung mit Benzaldehyd. Anschliessend führt man die 3,5,-Bistrifluormethylbenzoyl-Gruppe ein, beispielsweise wie für Verfahrensvariante a) beschrieben durch Kupplung mit einer Verbindung der Formel IIb, und spaltet die Schutzgruppe der 4-Aminogruppe ab, beispielsweise durch Behandeln mit einer Säure, wie Salzsäure, und gelangt so zu einer entsprechenden Verbindung der Formel IIIa.

Verbindungen der Formel lla können in an sich bekannter Weise hergestellt werden. Beispielsweise geht man von einer Verbindung der Formel IIIg aus und kuppelt diese, beispielsweise wie für Verfahrensvariante b) beschrieben, mit einer Verbindung der Formel IIIb in Gegenwart eines Kupplungsreagenzes und führt so die entsprechende Acyl-Gruppe ein. Man erhält so eine entsprechende Verbindung der Formel IIa.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen, Säuren bzw. Alkohole sind beispielsweise optisch aktive Alkaloidbasen, wie Strychnin, Cinchonin oder Brucin, oder D- oder L-(1-Phenyl)ethylamin, 3-Pipecolin, Ephedrin, Amphetamin und ähnliche synthetisch zugängliche Basen, optisch aktive Carbonoder Sulfonsäuren, wie Chinasäure oder D- oder L-Weinsäure, D- oder L-Di-otoluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)ethanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgenden Beispiele dienen zur Illustration der Erfindung; Temperaturen sind in Grad Celsius, Drucke in mbar angegeben. FD-MS = "Field Desorption Mass Spectroscopy".

### Beispiel 1: (2R,4S)-N-[1-(3,5-Bistrifluormethyl-benzoyl)-2-(4-chlorbenzyl)-piperidin-4-yl]-4-oxo-4H-1-benzopyran-2-carboxamid

Eine Lösung von 4.36g (2R,4S)-4-Amino-1-(3,5-bistrifluormethyl-benzoyl)-2-(4-chlorbenzyl)-piperidin in 200ml Methylenchlorid wird mit 3.77ml Triethylamin und 2.35g 4-oxo-4H-1-benzopyran-2-carbonsäurechlorid (hergestellt aus der entsprechenden Carbonsäure, z.B. Fa. Sigma, durch Umsetzung mit Thionylchlorid) versetzt und 4 Stunden bei 20 ° gerührt. Das Reaktionsgemisch wird mit 1N wässriger Salzsäure und anschliessend mit Sole und Wasser gewaschen, über Magnesiumsulfat getrocknet und eingedampft. Der aufgeschäumte Rückstand wird aus tert.Butyl-methylether/Hexan/Methylenchlorid kristallisiert. Die Titelverbindung wird so in Form von farblosen Kristallen vom Smp. 211-212° erhalten.
[α]_{D}²⁰= 3.7±2.6 (c=0.382, Methanol).

Die Ausgangsverbindung kann wie folgt hergestellt werden:
a) N-[1-(4-Chlor-benzyl)-but-3-enyl]-N-ethoxymethyl-carbaminsäuremethylester: Eine Suspension von 10.0 g Natriumhydrid (80% in Mineralöl, 333 mMol) in trockenem Tetrahydrofuran [THF] unter Argon wird auf Rückfluss erhitzt. Eine Lösung von 60.5 g (238 mMol) [1-(4-Chlor-benzyl)-but-3-enyl]-carbaminsäuremethylester [McCarty FJ et al., J. Med. Chem, 1968, 11 (3),534] in 50 ml trockenem THF wird innerhalb 1 Stunde zugetropft. Anschliessend wird das Gemisch noch 2 Stunden unter Rückfluss gekocht bis die Gasentwicklung nachlässt. Das Gemisch wird auf 0°C abgekühlt und Chlormethylethylether zugetropft, so dass die Reaktionstemperatur nicht über 5°C steigt. Nachher wird langsam auf 25°C erwärmt und 12 Stunden ausgerührt. Überschüssiges Natriumhydrid wird vorsichtig mit 1 ml Wasser vernichtet bevor man mehr Wasser zugibt. Die Phasen werden getrennt und die Wasserphase mit tert-Butylmethylether extrahiert. Die kombinierten organischen Phasen werden mit Sole gewaschen, über Natriumsulfat getrocknet und eingedampft. Das Rohprodukt wird bei 0.1 mbar destilliert und hat einen Siedebereich von 120-125°C. DC: Ethylacetat/Hexan (1:6) R_{f}= 0.34, FD-MS: M⁺= 311(313).
b) (2R*,4S*)-4-Acetylamino-2-(4-chlor-benzyl)-piperidin-1-carbonsäure-methylester: Bei -40°C werden 20.6 ml (308 mMol) Chlorsulfonsäure in 500 ml Acetonitril vorgelegt. Eine Lösung von 48.0 g (154 mMol) N-[1-(4-Chlor-benzyl)-but-3-enyl]-N-ethoxymethyl-carbaminsäuremethylester in 50 ml Acetonitril wird zugetropft, so dass die Reaktionstemperatur nicht über -10°C hinaussteigt. Anschliessend wird noch 20 Minuten bei -15°C gerührt, bevor man die Reaktion mit 370 ml 2N Natronlauge und 100 ml 10%-ige wässrige Natriumhydrogencarbonatlösung versetzt. Die Phasen werden getrennt und die wässrige Phase noch zweimal mit Toluol extrahiert. Die kombinierten organischen Phasen werden über Natriumsulfat getrocknet. Das Rohprodukt wird aus Toluol kristallisiert und ergibt die Titelverbindung als weisse Kristalle. Smp. 169-170°C. DC: Methylenchlorid/Methanol/konz. Ammoniak (90 : 9 :1) R_{f}= 0.42, FD-MS: M⁺= 325.
c) (2R*,4S*) N-[2-(4 -Chlor-benzyl)-piperidin-4-yl]-acetamid: (2R*,4S*)-4-Acetylamino-2-(4-chlor-benzyl)-piperidin-1-carbonsäure-methylester (30.0 g, 92.3 mMol) wird mit 51.8 ml 33 prozentigem Bromwasserstoff in Essigsäure versetzt. Nach 16 Stunden wird das Gemisch mit 200 ml Wasser versetzt und zweimal mit Toluol gewaschen. Die Wasserphase wird basisch gestellt und zweimal mit Essigsäureethylester extrahiert. Die organischen Phasen werden auf Kaliumcarbonat getrocknet und am Rotationsverdampfer eingedampft. Die Titelverbindung kristallisiert als Hydrochlorid aus Ethanol/Ethylacetat. Smp. 288-289°C. DC: Methylenchlorid/Methanol/konz. Ammoniak (90:9:1) R_{f}= 0.17, FD-MS: (M+1)⁺= 267.
d) (2'S,2R,4S)-Essigsäure-2-[4-acetylamino-2-(4-chlor-benzyl)-piperidin-1-yl]-2-oxo-1-phenyl-ethylester: Racemisches N-[2-(4-Chlor-benzyl)-piperidin-4-yl]-acetamid-hydrochlorid (20.5 g, 67.6 mMol) wird unter kräftigem Rühren bei 0°C in 34 ml 2N Natronlauge, 150 ml einer 10-prozentigen wässrigen Natriumhydrogencarbonatlösung und 50 ml Methylenchlorid vorgelegt. Man tropft während 1 Stunde. S(+)-O-Acetyl-mandelsäurechlorid [Pracejus G, Ann., 1959, 622, 10] (14.9 g, 70 mMol) dazu. Anschliessend wird 1 Stunde bei +4°C nachgerührt. Die Phasen werden getrennt, die organische Phase über Natriumsulfat getrocknet und am Rotationsverdampfer eingedampft. Die Titelverbindung wird als reines Diastereomer nach zweifachem Kristallisieren aus Methylenchlorid /tert-Butylmethylether isoliert. Smp. 209-211°C. DC: Methylenchlorid/lsopropanol (9:1) R_{f}= 0.65, FD-MS: M⁺= 443. [alpha]_{D} = + 77.5 Grad (c=1, Methylenchlorid).
   Die Mutterlaugen enthalten hauptsächlich das nicht-kristalline Diastereomer (2'S,2S,4R)N-[2-(4-Chlor-benzyl)-1-(acetoxy-phenyl-acetyl)piperidin-4-yl]acetamid, DC: Methylenchlorid/lsopropanol (9:1) R_{f}= 0.70.
e) (2R,4S)-4-Amino-1-(3.5-bistrifluormethyl-benzoyl)-2-(4-chlorbenzyl)-piperidin:
   (2'S,2R,4S)-Essigsäure-2-[4-acetylamino-2-(4-chlor-benzyl)-piperidin-1-yl]-2-oxo-1-phenylethyl-ester (37.4 g, 84.5 mMol) wird während 2 Tagen in 370 ml 6 N Salzsäure unter Rückfluss gekocht. Nach Abkühlen wird das Gemisch mit festem Natriumhydroxid basisch gestellt und mit Methylenchlorid extrahiert. Die kombinierten organischen Phasen werden über Kaliumcarbonat getrocknet und am Rotationsverdampfer eingedampft. Der Rückstand, bestehend aus fast reinem (2R,4S)-2-(4-Chlor-benzyl)-piperidin-4-amin (19.0 g, 84.5 mMol, 100%), wird mit 8.5 ml (84.5 mMol) Benzaldehyd versetzt und zwei Mal mit 150 ml Toluol am Rotationsverdampfer eingeengt. Der ölige Rückstand wird in 180 ml Methylenchlorid und 15.3 ml (110 mMol) Triethylamin aufgenommen und auf 10°C gekühlt. Bistrifluormethylbenzoylchlorid (25.7 g, 92.9 mMol) wird während 15 Minuten zugetropft und anschliessend 1 Stunde bei 25°C ausgerührt. Das Reaktionsgemisch wird mit 250 ml 1 N Salzsäure versetzt und das Methylenchlorid unter vermindertem Druck am Rotationsverdampfer entfernt. Hexan und Ethanol werden zugegeben bis zwei homogene Phasen entstehen. Nach Abtrennen der organischen Phase wird weiter mit Hexan gewaschen bis alles Benzaldehyd entfernt ist. Das Gemisch wird mit festem Natriumhydroxid basisch gestellt und wiederholt mit Methylenchlorid extrahiert. Die organischen Phasen werden über Natriumsulfat getrocknet und am Rotationsverdampfer eingeengt. Kristallisation aus tert-Butylmethylether/Hexan ergibt die Titelverbindung als weisse Kristalle. Smp. 79-81°C. DC: Methylenchlorid/Methanol/konz. Ammoniak (90:9:1) R_{f}= 0.21, FD-MS: (M+1)⁺= 465.
   [alpha]^{D} = -12.7 Grad (c=1, Methylenchlorid).

In analoger Weise, wie unter Beispiel 1 beschrieben, können auch die folgenden Verbindungen hergestellt werden. Die Herstellung der entsprechenden Ausgangsverbindung dafür, (2R,4S)-4-Amino-1-(3,5-bistrifluormethyl-benzoyl)-2-benzyl-piperidin, ist in EP-A-532 456, Beispiel 38f, beschrieben:

### Beispiel 1/1: (2R,4S)-N-[1-(3,5-Bistrifluormethyl-benzoyl)-2-benzyl-piperidin-4-yl]-4-oxo-4H-1-benzopyran-2-carboxamid, Smp. 107-108°, [α]_{D}²⁰=18.3±2.6 (c=0.388, Methanol)

### Beispiel 1/2: (2R,4S)-N-[1-(3,5-Bistrifluormethyl-benzoyl)-2-benzyl-piperidin-4-yl]-7-chlor-4-oxo-4H-1-benzopyran-2-carboxamid, Smp. 224-226°, [α]_{D}²⁰=21.5±2.5 (c=0.40, Methanol)

### Beispiel 1/3: (2R,4S)-N-[1-(3,5-Bistrifluormethyl-benzoyl)-2-benzyl-piperidin-4-yl]-7-methoxy-4-oxo-4H-1-benzopyran-2-carboxamid, Smp. 190-192°, [α]_{D}²⁰= 25.7±2.3 (c=0.44, Methanol)

### Beispiel 1/4: (2R,4S)-N-[1-(3,5-Bistrifluormethyl-benzoyl)-2-benzyl-piperidin-4-yl]-7-methylthio-4-oxo-4H-1-benzopyran-2-carboxamid

### Beispiel 1/5: (2R,4S)-N-[1-(3,5-Bistrifluormethyl-benzoyl)-2-benzyl-piperidin-4-yl]-6-methoxy-4-oxo-4H-1-benzopyran-2-carboxamid

### Beispiel 1/6: (2R,4S)-N-[1-(3,5-Bistrifluormethyl-benzoyl)-2-benzyl-piperidin-4-yl]-6-chlor-4-oxo-4H-1-benzopyran-2-carboxamid

### Beispiel 1/7: (2R,4S)-N-[1-(3,5-Bistrifluormethyl-benzoyl)-2-benzyl-piperidin-4-yl]-6-brom-4-oxo-4H-1-benzopyran-2-carboxamid

### Beispiel 1/8: (2R,4S)-N-[1-(3,5-Bistrifluormethyl-benzoyl)-2-benzyl-piperidin-4-yl]-6-fluor-4-oxo-4H-1-benzopyran-2-carboxamid

### Beispiel 1/9: (2R,4S)-N-[1-(3,5-Bistrifluormethyl-benzoyl)-2-benzyl-piperidin-4-yl]-6-methyl-4-oxo-4H-1-benzopyran-2-carboxamid

### Beispiel 1/10: (2R,4S)-N-[1-(3,5-Bistrifluormethyl-benzoyl)-2-benzyl-piperidin-4-yl]-6-cyano-4-oxo-4H-1-benzopyran-2-carboxamid

### Beispiel 1/11: (2R,4S)-N-[1-(3,5-Bistrifluormethyl-benzoyl)-2-benzyl-piperidin-4-yl]-6-nitro-4-oxo-4H-1-benzopyran-2-carboxamid

### Beispiel 1/12: (2R,4S)-N-[1-(3,5-Bistrifluormethyl-benzoyl)-2-benzyl-piperidin-4-yl]-7-fluor-4-oxo-4H-1-benzopyran-2-carboxamid

### Beispiel 1/13: (2R,4S)-N-[1-(3,5-Bistrifluormethyl-benzoyl)-2-benzyl-piperidin-4-yl]-7-brom-4-oxo-4H-1-benzopyran-2-carboxamid

### Beispiel 1/14: (2R,4S)-N-[1-(3,5-Bistrifluormethyl-benzoyl)-2-benzyl-piperidin-4-yl]-7-methyl-4-oxo-4H-1-benzopyran-2-carboxamid

### Beispiel 1/15: (2R,4S)-N-[1-(3,5-Bistrifluormethyl-benzoyl)-2-benzyl-piperidin-4-yl]-7-vitro-4-oxo-4H-1-benzopyran-2-carboxamid

### Beispiel 1/16: (2R,4S)-N-[1-(3,5-Bistrifluormethyl-benzoyl)-2-benzyl-piperidin-4-yl]-6,7-dimethoxy-4-oxo-4H-1-benzopyran-2-carboxamid.

### Beispiel 2: (2R,4S)-N-[1-(3.5-Bistrifluormethyl-benzoyl)-2-benzyl-piperidin-4-yl]-7-hydroxy-4-oxo-4H-1-benzopyran-2-carboxamid

Eine Lösung von 0.127g (2R,4S)-4-Amino-1-(3,5-bistrifluormethyl-benzoyl)-2-benzylpiperidin in 3.1ml Methylenchlorid wird mit 0.038g 4-Dimethylaminopyridin, 0.059g N-(3-Dimethylaminopropyl)-N'-ethyl-carbodiimid-hydrochlorid und 0.064g 7-hydroxy-4-oxo-4H-1-benzopyran-2-carbonsäure in 2ml Methylenchlorid/Dimethylformamid (1:1) versetzt und 24 Stunden bei 20° gerührt. Das Reaktionsgemisch wird eingedampft und der Rüchstand an Kieselgel mit Methylenchlorid und Methylenchlorid/Methanol (19:1) chromatographiert. Die Titelverbindung wird so als hellgelbes Pulver vom Smp. 224-225° erhalten; [α]_{D}²⁰= 23.3± 3.5 (c=0.288, Methanol).

In analoger Weise wie unter Beispiel 2 beschrieben, kann auch die folgende Verbindung hergestellt werden:

### Beispiel 2/1:(2R,4S)-N-[1-(3,5-Bistrifluormethyl-benzoyl)-2-benzyl-piperidin-4-yl]-6-brom-7-hydroxy-4-oxo-4H-1-benzopyran-2-carboxamid. Smp. 173-174°.

### Beispiel 3: In analoger Weise wie unter Beispiel 1 beschrieben können, ausgehend von (2R,4S)-4-Amino-1-(3,5-bistrifluormethyl-benzoyl)-2-(4-chlorbenzyl)-piperidin [Beispiel 1e)], auch die folgenden Verbindungen hergestellt werden:

### Beispiel 3/1: (2R,4S)-N-[1-(3,5-Bistrifluormethyl-benzoyl)-2-(4-chlorbenzyl)-piperidin-4-yl]-7-chlor-4-oxo-4H-1-benzopyran-2-carboxamid. Smp. 218-220°, [α]_{D}²⁰= 31.5±2.0 (c=0.50, Methanol).

### Beispiel 3/2: (2R,4S)-N-[1-(3,5-Bistrifluormethyl-benzoyl)-2-(4-chlorbenzyl)-piperidin-4-yl]-7-methoxy-4-oxo-4H-1-benzopyran-2-carboxamid. Smp. 198-200°, [α]_{D}²⁰= 29.7±2.2 (c=0.45, Methanol).

### Beispiel 3/3: (2R,4S)-N-[1-(3,5-Bistrifluormethyl-benzoyl)-2-(4-chlorbenzyl)-piperidin-4-yl]-7-methylthio-4-oxo-4H-1-benzopyran-2-carboxamid. Smp. 137-140°, [α]_{D}²⁰=20.8±2.8 (c=0.355, Methanol).

### Beispiel 3/4: (2R,4S)-N-[1-(3,5-Bistrifluormethyl-benzoyl)-2-(4-chlorbenzyl)-piperidin-4-yl]-6-methoxy-4-oxo-4H-1-benzopyran-2-carboxamid.

### Beispiel 3/5: (2R,4S)-N-[1-(3,5-Bistrifluormethyl-benzoyl)-2-(4-chlorbenzyl)-piperidin-4-yl]-6-chlor-4-oxo-4H-1-benzopyran-2-carboxamid.

### Beispiel 3/6: (2R,4S)-N-[1-(3,5-Bistrifluormethyl-benzoyl)-2-(4-chlorbenzyl)-piperidin-4-yl]-6-brom-4-oxo-4H-1-benzopyran-2-carboxamid.

### Beispiel 3/7: (2R,4S)-N-[1-(3,5-Bistrifluormethyl-benzoyl)-2-(4-chlorbenzyl)-piperidin-4-yl]-6-fluor-4-oxo-4H-1-benzopyran-2-carboxamid. Smp. 215-218°; R_{f} (Ethylacetat/Hexan 4:1) = 0.58. Das als Ausgangsmaterial benötigte Säurechlorid 6-Fluor-4-oxo-4H-1-benzopyran-2-carbonsäurechlorid ist z.B. in Chemical Abstracts: 96:52132w oder 88:106066w beschrieben und hat die CAS Reg.Nr. 65843-87-0.

### Beispiel 3/8: (2R,4S)-N-[1-(3,5-Bistrifluormethyl-benzoyl)-2-(4-chlorbenzyl)-piperidin-4-yl]-6-methyl-4-oxo-4H-1-benzopyran-2-carboxamid. Smp. 240-241°; R_{f} (Ethylacetat/Hexan 4:1) = 0.65.

### Beispiel 3/9: (2R,4S)-N-[1-(3,5-Bistrifluormethyl-benzoyl)-2-(4-chlorbenzyl)-piperidin-4-yl]-6-cyano-4-oxo-4H-1-benzopyran-2-carboxamid.

### Beispiel 3/10: (2R,4S)-N-[1-(3,5-Bistrifluormethyl-benzoyl)-2-(4-chlorbenzyl)-piperidin-4-yl]-6-nitro-4-oxo-4H-1-benzopyran-2-carboxamid.

### Beispiel 3/11: (2R,4S)-N-[1-(3,5-Bistrifluormethyl-benzoyl)-2-(4-chlorbenzyl)-piperidin-4-yl]-7-fluor-4-oxo-4H-1-benzopyran-2-carboxamid.

### Beispiel 3/12: (2R,4S)-N-[1-(3,5-Bistrifluormethyl-benzoyl)-2-(4-chlorbenzyl)-piperidin-4-yl]-7-brom-4-oxo-4H-1-benzopyran-2-carboxamid.

### Beispiel 3/13: (2R,4S)-N-[1-(3,5-Bistrifluormethyl-benzoyl)-2-(4-chlorbenzyl)-piperidin-4-yl]-7-methyl-4-oxo-4H-1-benzopyran-2-carboxamid.

### Beispiel 3/14: (2R,4S)-N-[1-(3,5-Bistrifluormethyl-benzoyl)-2-(4-chlorbenzyl)-piperidin-4-yl]-7-nitro-4-oxo-4H-1-benzopyran-2-carboxamid.

### Beispiel 3/15: (2R,4S)-N-[1-(3,5-Bistrifluormethyl-benzoyl)-2-(4-chlorbenzyl)-piperidin-4-yl]-6,7-dimethoxy-4-oxo-4H-1-benzopyran-2-carboxamid.

### Beispiel 4: Tabletten enthaltend je 50 mg Wirkstoff können wie folgt hergestellt werden:

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch'zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 5: Lacktabletten enthaltend je 100 mg Wirkstoff können wie folgt hergestellt werden:

| Zusammensetzung (für 1000 Lacktabletten) | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropyl-methylcellulose und des Schellacks in Methylenchlorid lackiert; Endgewicht der Lacktablette: 283 mg.

### Beispiel 6: Gelatinesteckkapseln enthaltend 100 mg Wirkstoff können z.B. wie folgt hergestellt werden:

| Zusammensetzung (für 1000 Kapseln) | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3-minütigem weiteren Mischen werden je 390 mg der erhaltenen Formulierung in Gelatinesteckkapseln der Grösse 0 abgefüllt.

### Beispiel 7: Eine treibmittelhaltige Inhalationssuspension enthaltend 0,1 Gewichtsprozent Wirkstoff:

| Zusammensetzung | Gewichtsprozent |
|---|---|
| Wirkstoff, mikronisiert | 0,1 |
| Sorbitantrioleat | 0,5 |
| Treibmittel A (Trichlortrifluorethan) | 4,4 |
| Treibmittel B (Dichlordifluormethan und | 15,0 |
| 1,2-Dichlortetrafluorethan) | 80,0 |

Der Wirkstoff wird unter Feuchtigkeitsausschluss mit Hilfe eines üblichen Homogenisators unter Zusatz des Sorbitantrioleats im Trichlortrifluorethan suspendiert und die Suspension in einen mit einem Dosierventil versehenen Aerosolbehälter abgefüllt. Der Behälter wird verschlossen und unter Druck mit dem Treibmittel B aufgefüllt.

## Patentansprüche

1. Verbindungen der Formel I, 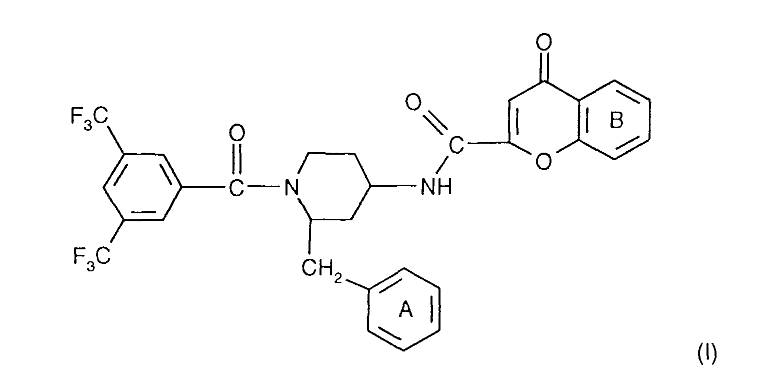 worin der Ring A unsubstituiert oder durch Niederalkyl, Niederalkoxy, Halogen, Nitro oder Trifluoromethyl monosubstituiert ist, und worin der Ring B unsubstituiert oder durch 1-4 Substituenten ausgewählt aus der Gruppe bestehend aus Niederalkyl, Hydroxy, Niederalkoxy, Niederalkylthio, Halogen, Nitro, Cyano und Trifluoromethyl substituiert ist, und worin der Ausdruck "nieder" bedeutet, dass entsprechende Gruppen und Verbindungen jeweils 1 bis und mit 7 C-Atome enthalten, und Salze davon.

2. Verbindungen der Formel I gemäss Anspruch 1, worin der Ring A unsubstituiert oder durch Halogen monosubstituiert ist, und worin der Ring B unsubstituiert oder durch 1-2 Substituenten ausgewählt aus der Gruppe bestehend aus Niederalkyl, Hydroxy, Niederalkoxy, Niederalkylthio, Halogen, Nitro und Cyano substituiert ist, und pharmazeutisch anwendbare Salze davon.

3. Verbindungen der Formel I gemäss Anspruch 1, worin der Ring A unsubstituiert oder durch Chlor monosubstituiert ist, und worin der Ring B unsubstituiert oder durch 1-2 Substituenten ausgewählt aus der Gruppe bestehend aus Hydroxy, Niederalkoxy, Chlor und Brom substituiert ist, und pharmazeutisch anwendbare Salze davon.

4. Verbindungen der Formel I gemäss Anspruch 1, worin der Ring A unsubstituiert oder durch Chlor monosubstituiert ist, und worin der Ring B unsubstituiert oder durch Chlor oder Fluor monosubstituiert ist, und pharmazeutisch anwendbaren Salze davon.

5. Verbindungen der Formel I gemäss einem der Ansprüche 1-4, wobei die Verbindungen der Formel I in der diastereomeren Form vorliegen, wie sie in Formel la wiedergegeben ist:

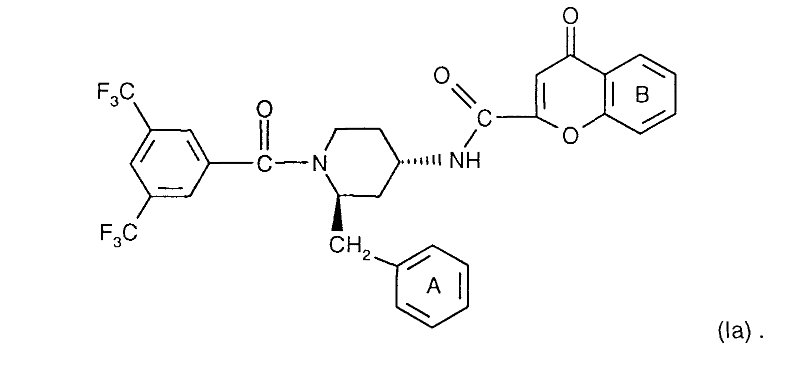

6. (2R,4S)-N-[1-(3,5-Bistrifluormethyl-benzoyl)-2-(4-chlorbenzyl)-piperidin-4-yl]-4-oxo-4H-1-benzopyran-2-carboxamid gemäss Anspruch 1 oder ein pharmazeutisch anwendbares Salz davon.

7. (2R,4S)-N-[1-(3,5-Bistrifluormethyl-benzoyl)-2-benzyl-piperidin-4-yl]-4-oxo-4H-1-benzopyran-2-carboxamid gemäss Anspruch 1 oder ein pharmazeutisch anwendbares Salz davon.

8. (2R,4S)-N-[1-(3,5-Bistrifluormethyl-benzoyl)-2-(4-chlorbenzyl)-piperidin-4-yl]-6-fluor-4-oxo-4H-1-benzopyran-2-carboxamid gemäss Anspruch 1 oder ein pharmazeutisch anwendbares Salz davon.

9. Pharmazeutische Präparate enthaltend eine Verbindung gemäss einem der Ansprüche 1 bis 8 und mindestens ein pharmazeutisch verwendbares Trägermaterial.

10. Eine Verbindung gemäss einem der Ansprüche 1 bis 8 zur Anwendung in einem Verfahren zur therapeutischen Behandlung des tierischen oder menschlichen Körpers.

11. Eine Verbindung gemäss einem der Ansprüche 1 bis 8 zur Verwendung bei der Behandlung von Krankheiten, die auf eine Antagonisierung des NK1-Rezeptors ansprechen.

12. Verwendung einer Verbindung gemäss einem der Ansprüche 1 bis 8 zur Herstellung pharmazeutischer Präparate.

13. Verwendung einer Verbindung gemäss einem der Ansprüche 1 bis 8 zur Herstellung pharmazeutischer Präparate für die Behandlung von Krankheiten, die auf eine Antagonisierung des NK1-Rezeptors ansprechen.

14. Verfahren zur Herstellung einer Verbindung der Formel I gemäss Anspruch 1, **dadurch gekennzeichnet, dass** man
a) eine Verbindung der Formel IIa, 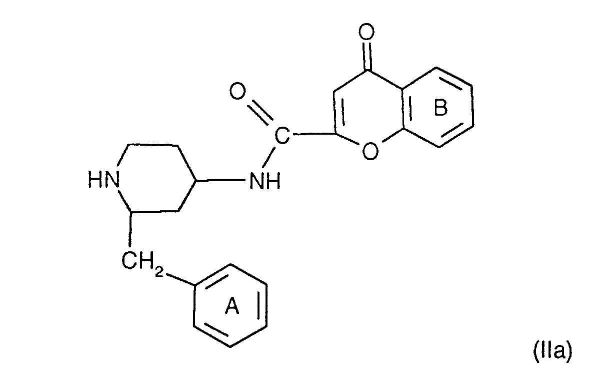 worin die Ringe A und B wie unter Formel I definiert sind, mit einer Verbindung der Formel IIb, 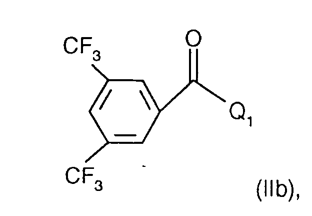 worin Q₁ gegebenenfalls verethertes Hydroxy, wie Hydroxy, C₁-C₇-Alkoxy oder gegebenenfalls substituiertes Phenoxy, oder reaktionsfähiges verestertes Hydroxy, wie Halogen, insbesondere Chlor, oder einen Rest der Formel 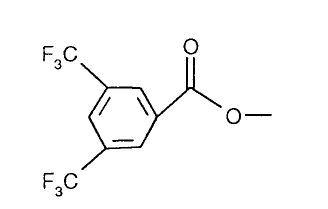 bedeutet, oder einem Salz davon, umsetzt; oder
b) eine Verbindung der Formel IIIa, 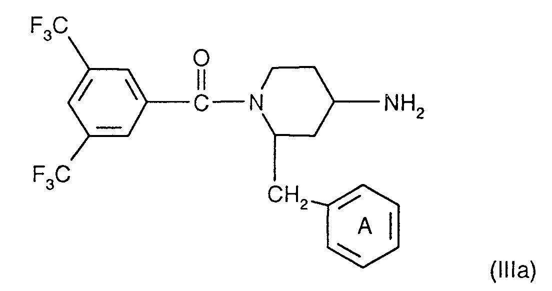
worin der Ring A wie unter Formel I angegeben definiert ist, mit einer Verbindung der Formel IIIb, 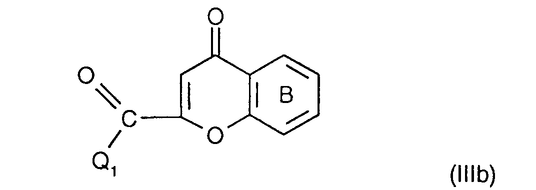 worin der Ring B wie unter Formel I angegeben definiert ist und Q₁ gegebenenfalls verethertes Hydroxy, wie Hydroxy, C₁-C₇-Alkoxy oder gegebenenfalls substituiertes Phenoxy, oder reaktionsfähiges verestertes Hydroxy, wie Halogen, insbesondere Chlor, oder einen Rest der Formel 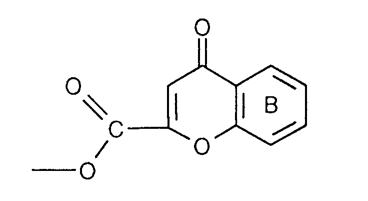 bedeutet, oder einem Salz davon, umsetzt;
und, wenn erwünscht, eine Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, eine erhaltene Mischung von Stereoisomeren oder Diastereomeren in die einzelnen Stereoisomeren bzw. Diastereomeren auftrennt.

## Claims

1. Compounds of formula I 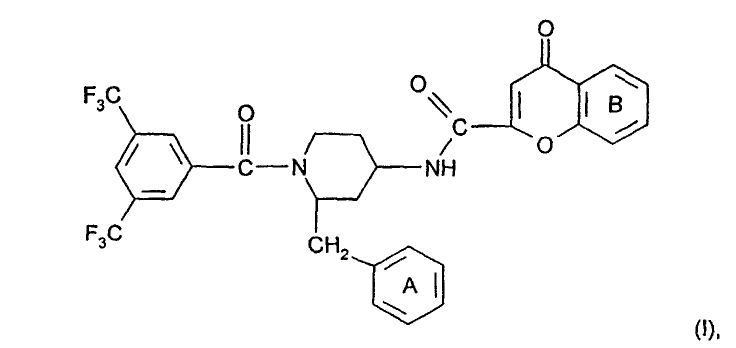 wherein ring A is unsubstituted or monosubstituted by lower alkyl, lower alkoxy, halogen, nitro or trifluoromethyl and ring B is unsubstituted or substituted by 1-4 substituents selected from the group consisting of lower alkyl, hydroxy, lower alkoxy, lower alkylthio, halogen, nitro, cyano and trifluoromethyl, and wherein the term "lower" means that corresponding groups and compounds each contain 1 up to and including 7 carbon atoms, and salts thereof.

2. Compounds of formula I according to claim 1, wherein ring A is unsubstituted or monosubstituted by halogen and ring B is unsubstituted or substituted by 1-2 substituents selected from the group consisting of lower alkyl, hydroxy, lower alkoxy, lower alkylthio, halogen, nitro and cyano, and pharmaceutically acceptable salts thereof.

3. Compounds of formula I according to claim 1, wherein ring A is unsubstituted or monosubstituted by chloro and ring B is unsubstituted or substituted by 1-2 substituents selected from the group consisting of hydroxy, lower alkoxy, chloro and bromo, and pharmaceutically acceptable salts thereof.

4. Compounds of formula I according to claim 1, wherein ring A is unsubstituted or monosubstituted by chloro and ring B is unsubstituted or monosubstituted by chloro or fluoro, and pharmaceutically acceptable salts thereof.

5. Compounds of formula I according to any one of claims 1-4, wherein the compounds of formula I are in the diastereoisomeric form as represented in formula la:

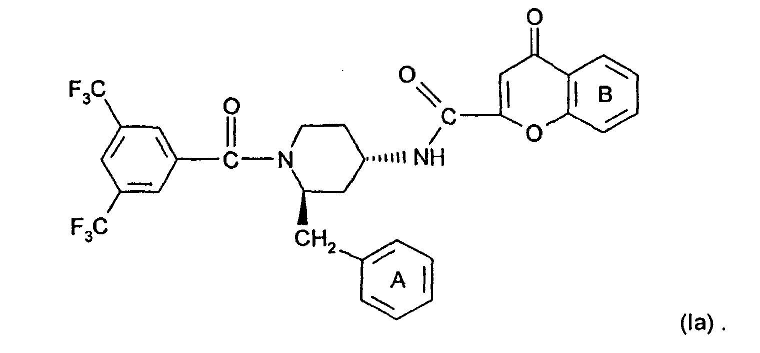

6. (2R,4S)-N-[1-(3,5-bistrifluoromethylbenzoyl)-2-(4-chlorobenzyl)piperidin-4-yl]-4-oxo-4H-1-benzopyrane-2-carboxamide according to claim 1 or a pharmaceutically acceptable salt thereof.

7. (2R,4S)-N-[1-(3,5-bistrifluoromethylbenzoyl)-2-benzylpiperidin-4-yl]-4-oxo-4H-1-benzopyrane-2-carboxamide according to claim 1 or a pharmaceutically acceptable salt thereof.

8. (2R,4S)-N-[1-(3,5-blstrifluoromethylbenzoyl)-2-(4-chlorobenzyl)piperidin-4-yl]-6-fluoro-4-oxo-4H-1-benzopyrane-2-carboxamide according to claim 1 or a pharmaceutically acceptable salt thereof.

9. A pharmaceutical composition comprising a compound according to any one of claims 1 to 8 and at least one pharmaceutically acceptable carrier.

10. A compound according to any one of claims 1 to 8 for use in a process for the therapeutic treatment of the animal or human body.

11. A compound according to any one of claims 1 to 8 for use in the treatment of diseases responsive to an antagonisation of the NK1 receptor.

12. Use of a compound according to any one of claims 1 to 8 for the preparation of pharmaceutical compositions.

13. Use of a compound according to any one of claims 1 to 8 for the preparation of pharmaceutical compositions for the treatment of diseases responsive to an antagonisation of the NK1 receptor.

14. A process for the preparation of a compound of formula I according to claim 1, which comprises
a) reacting a compound of formula IIa 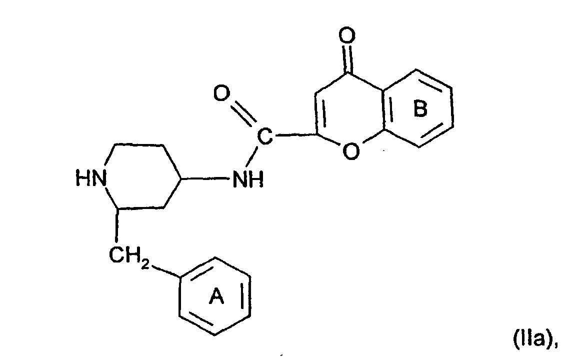 wherein rings A and B are as defined for formula I, with a compound of formula IIb 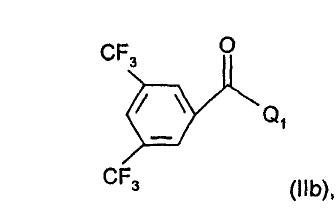 wherein Q₁ is hydroxy which may be etherified, e.g. hydroxy, C₁-C₇-alkoxy or unsubstituted or substituted phenoxy, or reactive esterified hydroxy, e.g. halogen, preferably chloro, or a radical of formula 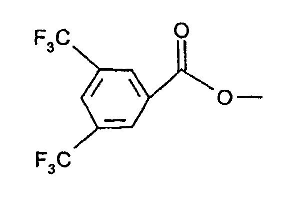 or a salt thereof; or
b) reacting a compound of formula IIIa 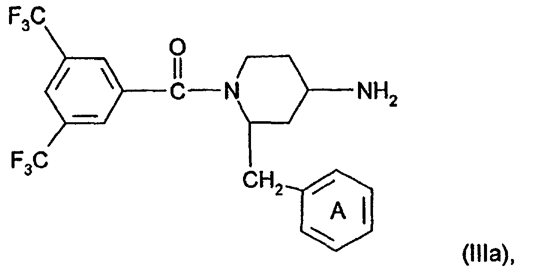
wherein ring A is as defined for formula I, with a compound of formula IIIb 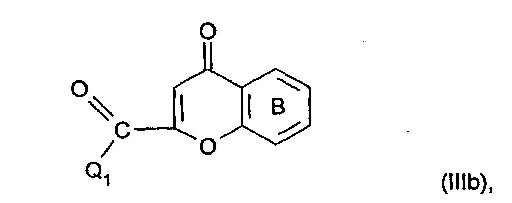 wherein ring B is as defined for formula I and Q₁ is hydroxy which may be etherified, e.g. hydroxy, C₁-C₇-alkoxy or unsubstituted or substituted phenoxy, or reactive esterified hydroxy, e.g. halogen, preferably chloro, or a radical of formula 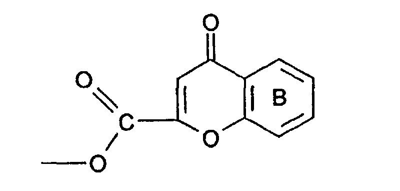 or a salt thereof;
and, if desired, converting a compound of formula I into another compound of formula I and/or, if desired, converting a salt obtained into the free compound or into another salt and/or, if desired, converting a free compound of formula I obtained having salt-forming properties into a salt and/or, if desired, separating a mixture of stereoisomers or diastereoisomers obtained into the individual stereoisomers and diastereoisomers.

## Revendications

1. Composés de formule I, 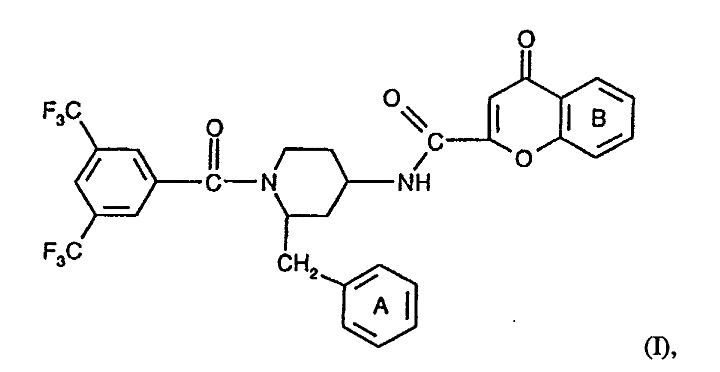 dans laquelle le cycle A est non substitué ou est monosubstitué par un alkyle inférieur, un alcoxy inférieur, un halogène, un nitro ou un trifluorométhyle, et dans laquelle le cycle B est non substitué ou est substitué par 1 à 4 substituants choisis dans le groupe composé d'un alkyle inférieur, d'un hydroxy, d'un alcoxy inférieur, d'un alkylthio inférieur, d'un halogène, d'un nitro, d'un cyano et d'un trifluorométhyle, et dans laquelle l'expression "inférieur(e)" signifie que les groupes et composés correspondants contiennent respectivement 1 à 7, inclus, atomes de carbone, et leurs sels.

2. Composés de formule I selon la revendication 1, dans lesquels le cycle A est non substitué ou est monosubstitué par un halogène, et dans lesquels le cycle B est non substitué ou est substitué par 1 ou 2 substituants choisis dans le groupe composé d'un alkyle inférieur, d'un hydroxy, d'un alcoxy inférieur, d'un alkylthio inférieur, d'un halogène, d'un nitro et d'un cyano, et leurs sels acceptables sur le plan pharmaceutique.

3. Composés de formule I selon la revendication 1, dans lesquels le cycle A est non substitué ou est monosubstitué par un chlore, et dans lesquels le cycle B est non substitué ou est substitué par 1 ou 2 substituants choisis dans le groupe composé d'un hydroxy, d'un alcoxy inférieur, d'un chlore et d'un brome, et leurs sels acceptables sur le plan pharmaceutique.

4. Composés de formule I selon la revendication 1, dans lesquels le cycle A est non substitué ou est monosubstitué par un chlore, et dans lesquels le cycle B est non substitué ou est monosubstitué par un chlore ou un fluor, et leurs sels acceptables sur le plan pharmaceutique.

5. Composés de formule I selon l'une des revendications 1 à 4, dans lesquels les composés de formule I se présentent sous la forme de diastéréomères, tels que reflétés par la formule Ia:

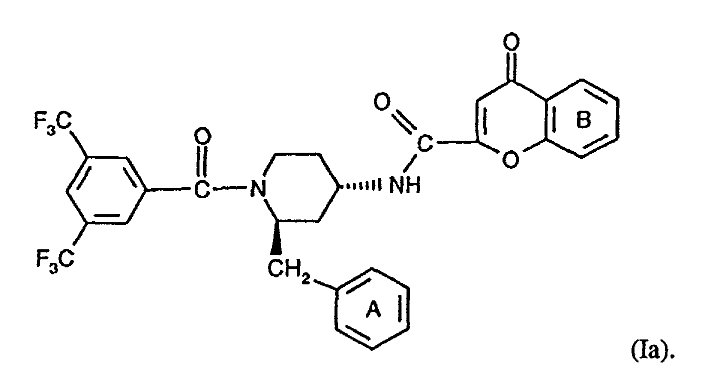

6. (2R, 4S)-N-[1-(3,5-bistrifluorométhyl-benzoyl)-2-(4-chlorobenzyl)-pipéridin-4-yl]-4-oxo-4H-1-benzopyran-2-carboxamide selon la revendication 1, ou un de ses sels acceptables sur le plan pharmaceutique.

7. (2R, 4S)-N-[1-(3,5-bistrifluorométhyl-benzoyl)-2-benzylpipéridin-4-yl]-4-oxo-4H-1-benzopyran-2-carboxamide selon la revendication 1, ou un de ses sels acceptables sur le plan pharmaceutique.

8. (2R, 4S)-N-[1-(3,5-bistrifluorométhyl-benzoyl)-2-(4-chlorobenzyl)-pipéridin-4-yl]-6-fluor-4-oxo-4H-1-benzopyran-2-carboxamide selon la revendication 1, ou un de ses sels acceptables sur le plan pharmaceutique.

9. Préparations pharmaceutiques contenant un composé selon l'une des revendications 1 à 8 et au moins un matériau de support pouvant être utilisé sur le plan pharmaceutique.

10. Composé selon l'une des revendications 1 à 8 à utiliser dans un procédé de traitement thérapeutique du corps animal ou humain.

11. Composé selon l'une des revendications 1 à 8 à utiliser dans le traitement de maladies qui réagissent à une antagonisation du récepteur NK1.

12. Utilisation d'un composé selon l'une des revendications 1 à 8 pour fabriquer des préparations pharmaceutiques.

13. Utilisation d'un composé selon l'une des revendications 1 à 8 pour fabriquer des préparations pharmaceutiques destinées au traitement de maladies qui réagissent à une antagonisation du récepteur NK1.

14. Procédé de fabrication d'un composé de formule I selon la revendication 1, **caractérisé en ce que** l'on fait réagir
a) un composé de formule IIa, 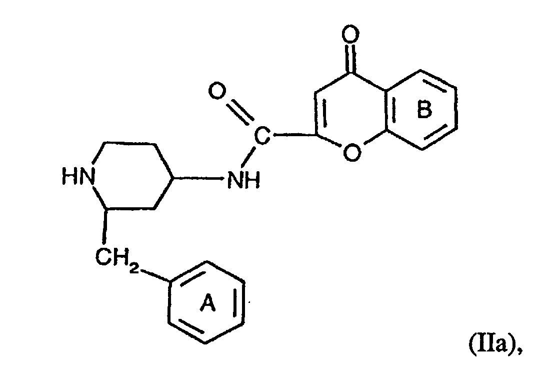 dans laquelle les cycles A et B sont définis tels que sous la formule I, avec un composé de formule IIb, 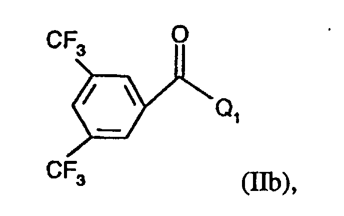 dans laquelle Q₁ est un hydroxy éventuellement éthérifié, comme un hydroxy, un alcoxy en C₁ à C₇ ou un phénoxy éventuellement substitué, ou un hydroxy estérifié réactif, comme un halogène, en particulier le chlore, ou un radical de formule 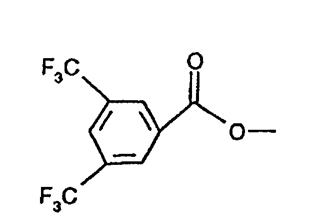 ou un de ses sels ; ou
on fait réagir
b) un composé de formule IIIa, 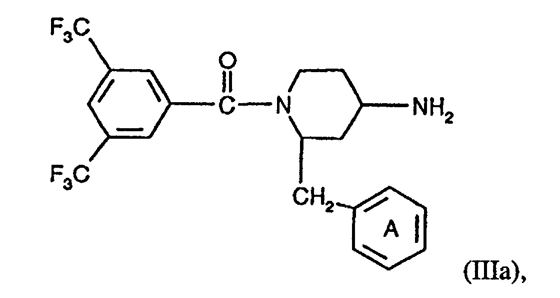
dans laquelle le cycle A est tel que défini sous la formule I, avec un composé de formule IIIb, 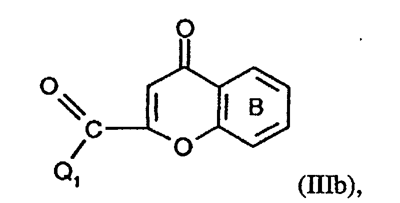 dans laquelle le cycle B est tel que défini sous la formule I, et Q₁ est un hydroxy éventuellement éthérifié, comme un hydroxy, un alcoxy en C₁ à C₇ ou un phénoxy éventuellement substitué, ou un hydroxy estérifié réactif, comme un halogène, en particulier le chlore, ou un radical de formule 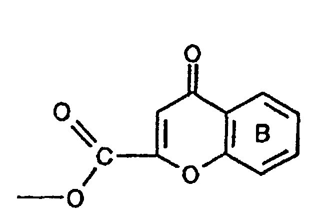 ou un de ses sels ;
et, si cela est souhaité, on convertit un composé de formule I en un autre composé de formule I et/ou, si cela est souhaité, on convertit un sel obtenu en son composé libre ou en un autre sel, et/ou si cela est souhaité, on convertit un composé libre obtenu de formule I présentant des propriétés salifiables en un sel, et/ou, si cela est souhaité, on sépare un mélange obtenu de stéréoisomères ou de diastéréomères en stéréoisomères ou diastéréomères distincts.
